# EUROPÄISCHE PATENTSCHRIFT

(11) **EP 1 677 697 B1**
(45) Veröffentlichungstag und Bekanntmachung des Hinweises auf die Patenterteilung: **16.09.2009**
(21) Anmeldenummer: 04790758.9
(22) Anmeldetag: 22.10.2004
(51) Int. Cl.: A61B 19/00, A61B 17/04

(54) **VORRICHTUNG ZUM FIXIEREN UND SPANNEN ZUMINDEST EINES ZUGFADENS FÜR DAS ANLEGEN EINER NEOVAGINA**
DEVICE FOR FIXING AND TENSIONING AT LEAST ONE PULLING THREAD FOR APPLYING A NEOVAGINA
DISPOSITIF DE FIXATION ET DE TENSION AU MOINS D'UN FIL DE TRACTION POUR L'APPLICATION D'UN NEOVAGIN

(30) Priorität: 24.10.2003 DE 10349953
(43) Veröffentlichungstag der Anmeldung: 12.07.2006
(73) Patentinhaber: Karl Storz GmbH & Co. KG, 78532 Tuttlingen (DE)
(72) Erfinder: DOLL, Frank, 78607 Talheim (DE); WALTER, Christian, 78576 Emmingen-Liptingen (DE)
(74) Vertreter: Heuckeroth, Volker
(86) Internationale Anmeldenummer: PCT/EP2004/011965
(87) Internationale Veröffentlichungsnummer: WO 2005/039429

(56) Entgegenhaltungen:
- EP-A- 0 615 725
- WO-A1-99/35974
- US-A- 3 650 274
- US-A- 5 649 960
- US-A- 6 120 525

## Beschreibung

Die Erfindung betrifft eine Vorrichtung zum Fixieren und Spannen zumindest eines Zugfadens für das Anlegen einer Neovagina, mit einem Grundkörper, an dem zumindest ein Fixierelement zum Fixieren des zumindest einen Zugfadens unter Spannung angeordnet ist, wobei das Fixierelement zum Spannen des zumindest einen Zugfadens um eine Drehachse drehbar ist, wobei eine Feststelleinrichtung für das Fixierelement vorhanden ist, mittels der das Fixierelement zumindest in einer Drehrichtung, die der Drehrichtung zum Spannen des zumindest einen Zugfadens entgegengerichtet ist, feststellbar ist.

Eine Vorrichtung mit den zuvor genannten Merkmalen zum Fixieren und Spannen zumindest eines zugfadens, jedoch zur verwendung zum Spannen von Haut zum Schließen von Hautwunden ist aus dem Dokument US-A-6 120 525 bekannt.

Diese Hautspannvorrichtung weist ein Ankerelement mit einem Körperabschnitt und einem Paar von ersten Fadenführungsachlitzen auf. Das Ankerelement wird auf einer Seite einer Wunde positioniert. Eine Winde ist über Fäden mit dem Ankerelement gekoppelt. Die Winde weist einen Windenkörper auf, der ein Paar zweiter Fadenführungsschlitze aufweist. Der windenkörper wird auf einer gegenüberliegenden Seite der Wunde positioniert, und der Faden wird durch die ersten und zweiten Fadenführungsschlitze des Ankerelements und Windenkörpers gefädelt, so dass sich der Faden auch durch die Haut unterhalb der Vorrichtung und durch die Wunde hindurch erstreckt. Die Winde ist mit einem Rastgesperre ausgebildet.

Eine Vorrichtung zum Fixieren und Spannen zumindest eines Zugfadens für das Anlegen einer Neovagina ist aus dem DE-Fachartikel von J. Keckstein, "Anlegen einer Neovagina (nach vecchietti)", in: "Die endoskopischen Operationen in der Gynäkologie", Herausgeber: J. Keckstein, Hucke, Urban und Fischer Verlag 2001, 1. Auflage, Seiten 332-338 bekannt.

Eine Vorrichtung der eingangs genannten Art wird im Rahmen eines operativen Verfahrens zum Anlegen einer Neovagina verwendet. Unter dem Anlegen einer Neovagina wird eine Scheidenrekonstruktion verstanden, die beim Fehlen einer Scheidenanlage vorgenommen wird, das eine Fehlbildung des weiblichen Genitales ist, die beim Mayer-Rokitansky-Küster-Syndrom und bei testikulärer Feminisierung anzutreffen ist.

Das operative Verfahren zur Scheidenrekonstruktion wurde ursprünglich laparotomisch und später laparoskopisch, d.h. auf endoskopischem Wege, durchgeführt.

Das operative Prinzip zum Anlegen einer Neovagina besteht in einer Dehnung des Vaginalgrübchens. Über eine Kunststoffolive oder ein Steckphantom, das beispielsweise in dem Dokument 36 16 957 A1 beschrieben ist und das mit zwei Zugfäden verbunden ist, wird kontinuierlich Druck auf das Vaginalgrübchen ausgeübt und dadurch innerhalb von Tagen eine Neovagina gedehnt bzw. gebildet. Die beiden Zugfäden der Kunststoffolive bzw. des Steckphantoms werden vom Vaginalgrübchen aus mit einer geraden Ahle, mit deren Hilfe das Vaginalgrübchen perforiert wird, nach intraperitoneal, d. h. in den Bauchraum, gebracht und dann mit einer gebogenen Ahle retroperitoneal vor die Bauchdecke gezogen. Vor der Bauchdecke werden die beiden Zugfäden dann mit einer Vorrichtung der eingangs genannten Art fixiert und gespannt, so dass die Zugfäden auf die Kunststoffolive bzw. das Steckphantom eine kontinuierliche Zugkraft ausüben, wodurch die Olive bzw. das Steckphantom dann einen kontinuierlichen Dehnungsdruck auf das Vaginalgrübchen ausübt.

Die aus dem eingangs genannten DE-Fachartikel bekannte Vorrichtung zum Fixieren und Spannen der beiden Zugfäden weist einen in etwa rechteckigen flachen Grundkörper auf, an dessen beiden Schmalseiten jeweils ein Fixierelement für jeweils einen der beiden Zugfäden angeordnet ist. Jedem Fixierelement ist eine Feder zugeordnet, an der der entsprechende Zugfaden eingehängt ist. Die Federn haben die Funktion, plötzliche, durch körperliche Bewegung verursachte Zugkraftänderungen abzudämpfen. Die beiden Fixierelemente bestehen aus Drehschrauben, mittels der die Enden der Zugfäden eingeklemmt und somit fixiert werden. Mittels der Fixierelemente ist kein Spannen der Zugfäden möglich, vielmehr müssen die Zugfäden dieser Vorrichtung bei gelockerter Drehschraube von Hand angezogen werden, wobei vor jedem Nachspannen zunächst die Drehschraube des Fixierelements gelöst und der Zugfaden von Hand angezogen und danach die Drehschraube wieder zur Fixierung des Zugfadens angezogen wird. Somit ist das Spannen und Fixieren der Zugfäden relativ zeitaufwendig und umständlich.

Im Rahmen des Verfahrens des Anlegens der Neovagina ist jedoch ein häufiges Nachspannen der Zugfäden erforderlich, da die Kunststoffolive bzw. das Steckphantom ja kontinuierlich Druck auf das Vaginalgrübchen ausüben und dieses dabei dehnen soll.

Ein weiterer Nachteil dieser bekannten Vorrichtung besteht darin, dass für jeden Zugfaden ein separates Fixierelement vorhanden ist, so dass die beiden Zugfäden nacheinander und unabhängig voneinander gespannt und fixiert werden müssen. Dies hat nicht nur einen erhöhten Zeitaufwand beim Spannen und Fixieren der Zugfäden zur Folge, sondern auch ein gleichmäßiges Spannen der beiden Zugfäden ist erschwert. Eine gleichmäßige Zugspannung der beiden Zugfäden ist jedoch wichtig, da bei ungleichmäßiger Zugspannung nur einer der Zugfäden eine Zugkraft ausübt und somit eher reißen kann, aber auch die Zugkraftrichtung auf die Olive ist unter Umständen nicht optimal, wenn nur ein Faden zieht.

In demselben DE-Fachartikel ist eine weitere Vorrichtung zum Fixieren und Spannen der beiden Zugfäden beschrieben. Diese bekannte Vorrichtung besteht aus zwei getrennten Fixierelementen, d.h. diese sind nicht auf einem gemeinsamen Grundkörper angeordnet und befestigt. Jedes Fixierelement besteht aus einer kreisförmigen Grundplatte, auf der eine Schraubenfeder mit beabstandeten Windungen angeordnet ist, an die sich oben eine weitere kreisförmige Platte anschließt, auf der eine Fixierschraube zum Fixieren eines Zugfadens angeordnet ist. Auch mit diesen Fixierelementen muss das Spannen des jeweiligen Zugfadens durch Ziehen und Halten des Endes des Zugfadens in der Hand und anschließendes Festschrauben der Fixierschraube durchgeführt werden, was wie bei der zuvor beschriebenen bekannten Vorrichtung zeitaufwendig und umständlich ist. Darüber hinaus besteht bei dieser bekannten Vorrichtung der bereits zuvor erwähnte Nachteil, dass beide Zugfäden nicht simultan gespannt werden können, wodurch ein gleichmäßiges Spannen der beiden Zugfäden ebenfalls nicht kontrolliert möglich ist.

Aus dem Dokument US-A-3 650 274 ist des Weiteren eine Vorrichtung zum Kontrollieren und Verteilen der Spannung von chirurgischen Nähten in der Abdominalchirurgie bekannt. Diese Vorrichtung weist ein Element auf, das auf der Körperoberfläche zum Überbrücken der chirurgischen Inzision angeordnet wird und zum Spannen eines Zugfadens dient, der quer zur Inzision verläuft. Zum Spannen des Zugfadens weist die Vorrichtung ein drehbares Fixierelement auf.

Eine Nahtverankerungsvorrichtung zum Verankern einer Naht in Gewebe ist aus dem Dokument EP-A-0 615 725 bekannt. Diese Vorrichtung weist eine Drehspule zum Spannen eines Zugfadens auf, wobei die Drehspule über ein Rastgesperre feststellbar ist.

Eine weitere chirurgische Fadenspannvorrichtung zum Schließen von Wunden ist aus dem Dokument WO-A-99/35974 bekannt. Zum Messen der Fadenspannung, die am Wundverschlussfaden anliegt, wird über eine Feder gemessen.

Schließlich offenbart auch das Dokument US-A-5 649 960 eine Fadenspannvorrichtung zum Verschließen von Hautwunden.

Der Erfindung liegt die Aufgabe zugrunde, eine Vorrichtung zum Fixieren und Spannen zumindest eines Zugfadens für das Anlegen einer Neovagina der eingangs genannten Art dahingehend weiterzubilden, dass durch körperliche Bewegungen verursachte Zugkraftänderungen abgedämpft werden.

Erfindungsgemäß wird diese Aufgabe hinsichtlich der eingangs genannten Vorrichtung dadurch gelöst, dass dem Fixierelement eine Feder zugeordnet ist, deren erstes Ende am Grundkörper festgelegt ist, und mit deren zweitem Ende der Zugfaden beweglich, verbindbar ist, so daß Zugfaden zum Fixierelement hin umgelenkt wird.

Bei der erfindungsgemäßen Vorrichtung ist vorgesehen, dass das Fixierelement nicht nur dem Fixieren des zumindest einen Zugfadens unter Spannung dient, sondern auch dem Spannen des Zugfadens, indem das Fixierelement um eine Drehachse drehbar ist, wodurch sich der Zugfaden zum Spannen um einen Abschnitt des Fixierelements aufwickeln kann. Zum Fixieren und Spannen des zumindest einen Zugfadens wird demnach bei der erfindungsgemäßen Vorrichtung umgekehrt zu den bekannten Vorrichtungen vorgegangen. zunächst wird nämlich bei der erfindungsgemäßen Vorrichtung der Zugfaden am Fixierelement festgelegt bzw. fixiert und anschließend durch Drehen des Fixierelements gespannt.

Weiterhin ist bei der erfindungsgemäßen Vorrichtung vorgesehen, dass das Fixierelement zumindest in einer Drehrichtung, die der Drehrichtung zum Spannen des Zugfadens entgegengerichtet ist, mittels einer Feststelleinrichtung feststellbar ist. Wenn also durch Drehen des Fixierelements die gewünschte Spannung des Zugfadens erreicht ist, wird dann zur Aufrechterhaltung dieser Spannung die Drehbeweglichkeit des Fixierelements zumindest in der Drehrichtung, die der Drehrichtung zum Spannen des Zugfadens entgegengerichtet ist, blockiert.

Für das von Zeit zu Zeit notwendige Nachspannen des zumindest einen Zugfadens muss das Fixierelement lediglich in der Drehrichtung des Spannens weitergedreht werden, wodurch nicht nur das erstmalige Spannen, sondern auch das Nachspannen des zumindest einen Zugfadens sehr leicht zu betätigen ist. Aber auch ein Entspannen des Zugfaden im Fall, dass die Spannung des zugfadens zu hoch eingestellt wurde, lässt sich sehr leicht bewerkstelligen, indem nämlich die Feststelleinrichtung gelöst wird, wodurch sich das Fixierelement in der Drehrichtung, die der Drehrichtung zum Spannen des Zugfadens entgegengerichtet ist, zum Entspannen des Zugfadens drehen lässt.

Dem Fixierelement ist eine Feder zugeordnet, deren erstes Ende am Grundkörper festgelegt ist und deren zweites Ende zur beweglichen Verbindung mit dem Zugfaden und dessen Umlenkung zum Fixierelement hin ausgebildet ist.

Diese an sich bekannte Maßnahme hat den Vorteil, dass die Feder nicht nur eine gleichmäßige Spannung des zumindest einen zugfadens und Vorspannung desselben gewährleistet, sondern auch durch körperliche Bewegung verursachte zugkraftänderungen abdämpft.

Die erfindungsgemäße Vorrichtung ermöglicht somit ein sehr leicht handhabbares und wenig zeitaufwendiges Fixieren und Spannen des zumindest einen Zugfadens, darüber hinaus insbesondere auch ein sehr gut kontrollierbares Einstellen der Spannung des Zugfadens.

In einer bevorzugten Ausgestaltung weist die Feststelleinrichtung ein Rastgesperre zum Feststellen des Fixierelements in der zumindest einen Drehrichtung auf.

Mit dieser Maßnahme wird eine konstruktiv vorteilhaft einfache Feststelleinrichtung zum Feststellen des Fixierelement geschaffen, die darüber hinaus auch hinsichtlich ihrer Handhabung besonders einfach ist.

In diesem Zusammenhang ist es bevorzugt, wenn das Rastgesperre gegen unerwünschtes Entsperren verriegelbar ist.

Da die Vorrichtung bei ihrer Anwendung beim Anlegen einer Neovagina über einen langen Zeitraum auf dem Bauch der Patientin platziert ist, wird durch diese Maßnahme vorteilhafterweise vermieden, dass sich das Rastgesperre beispielsweise im Schlaf der Patientin versehentlich entriegelt, wodurch ein unerwünschtes Entspannen des zumindest einen Zugfadens sicher vermieden werden kann.

In einer weiteren bevorzugten Ausgestaltung weist das Fixierelement ein umfänglich gezahntes Rad auf, das zum Feststellen des Fixierelements mit einem mit der Verzahnung des Rads in Eingriff bringbaren ersten Sperrelement zusammenwirkt.

Der Vorteil dieser Ausgestaltung des Fixierelements hat den Vorteil, dass mit nur wenigen Bauteilen und somit geringem Kostenaufwand eine Feststelleinrichtung für das Fixierelement in Form eines Rastgesperres geschaffen wird. Das Sperrelement kann beispielsweise in Form eines Zapfens oder Stiftes ausgebildet sein, der mit der Verzahnung des Rades des Fixierelements durch Verschieben in und außer Eingriff bringbar ist.

In diesem Zusammenhang ist es bevorzugt, wenn das erste Sperrelement feststellbar ist, wenn es mit der Verzahnung des Rades in Eingriff steht.

Durch die Feststellbarkeit des ersten Sperrelements, wenn dieses mit der Verzahnung des Rades in Eingriff steht, wird auf konstruktiv vorteilhaft einfache Weise eine Maßnahme geschaffen, um ein unerwünschtes Entsperren des Rastgesperres zu vermeiden.

In einer weiteren damit in Zusammenhang stehenden Ausgestaltung ist zum Feststellen des ersten Sperrelements ein zweites Sperrelement vorhanden, das in den Bewegungsweg des ersten Sperrelements verstellbar ist.

Durch diese Maßnahme wird auf konstruktiv einfache Weise und hinsichtlich der Funktion betriebssichere Verriegelung des Rastgesperres gegen ein unerwünschtes Entsperren geschaffen.

In einer weiteren bevorzugten Ausgestaltung weist das Fixierelement einen geschlitzten Abschnitt zur Aufnahme eines Abschnitts des zumindest einen Zugfadens und eine Hülse zum Festklemmen des Zugfadens am Fixierelement auf.

Diese Maßnahme hat den Vorteil, dass das Festlegen des zumindest einen Zugfadens am Fixierelement auf sehr leicht handhabbare Weise vorgenommen werden kann, indem ein Abschnitt (üblicherweise das Ende) des Zugfadens in den Schlitz eingelegt und anschließend die Hülse auf den geschlitzten Abschnitt des Fixierelements beispielsweise aufgesteckt oder aufgeschraubt wird, wodurch dann der Zugfaden am Fixierelement festgeklemmt wird. Besonders bevorzugt ist es, wenn die Hülse auf den geschlitzten Abschnitt des Fixierelements aufsteckbar ist, da das Aufstecken sehr schnell durchführbar ist. Im Falle einer aufsteckbaren Hülse ist diese mit dem geschlitzten Abschnitt des Fixierelements vorzugsweise drehfest verbindbar, beispielsweise verrastbar.

Dabei ist es weiterhin bevorzugt, wenn die Hülse ein Stellrad zum Drehen des Fixierelements aufweist.

Hierbei ist von Vorteil, dass die Hülse gleichzeitig das Bedienelement zum Spannen des zumindest einen Zugfadens darstellt und somit die Anzahl der Bauteile der erfindungsgemäßen Vorrichtung weiter reduziert wird.

Weiterhin ist es bevorzugt, wenn am zweiten Ende der Feder eine bewegliche Führungsrolle angeordnet ist.

Da der Zugfaden sich durch die zuvor erwähnten zugkraftänderungen auf Grund einer körperlichen Bewegung der Patientin am zweiten Ende der Feder relativ zu diesem bewegen kann, wird durch diese Maßnahme vorteilhafterweise eine Reibung zwischen dem Zugfaden und dem zweiten Ende der Feder erheblich vermindert, wodurch ein Reißen des Zugfadens auf Grund eines Abriebes vermieden wird.

In einer weiteren bevorzugten Ausgestaltung ist am Grundkörper eine Zuführstelle für den Zugfaden vorhanden.

Durch das Vorsehen einer Zuführstelle für den Zugfaden am Grundkörper wird eine wohl definierte und gleichbleibende Zugkraftrichtung des Zugfadens gewährleistet. Die Zuführstelle kann beispielsweise in Form einer Fadenführung für den Zugfaden ausgebildet sein.

In einer weiteren bevorzugten Ausgestaltung ist die Zuführstelle bezüglich der Feder und dem Fixierelement so angeordnet, dass der Zugfaden von der Zuführstelle zunächst an dem Fixierelement vorbei zu dem zweiten Ende der Feder und von dort zum Fixierelement verläuft.

In diesem Zusammenhang ist es besonders bevorzugt, wenn die Zuführstelle bezüglich der Feder und dem Fixierelement so angeordnet ist, dass der Zugfaden an dem zweiten Ende um etwa 180° zu dem Fixierelement hin umgelenkt wird.

In einer weiteren bevorzugten Ausgestaltung ist an der Zuführstelle eine bewegliche Führungsrolle vorhanden.

Auch hier ist wieder wie bei der beweglichen Rolle an der Feder die Reibung des zumindest einen Zugfadens an der Zuführstelle vorteilhaft vermindert, wodurch ein Reißen des Zugfadens auf Grund Abriebes vermieden wird.

In einer weiteren, besonders bevorzugten Ausgestaltung ist das Fixierelement zum gemeinsamen Fixieren und Spannen des zumindest einen Zugfadens und zumindest eines weiteren Zugfadens ausgelegt.

Diese Maßnahme hat nun den besonderen Vorteil, dass mit der erfindungsgemäßen Vorrichtung die üblicherweise zwei an der Kunststoffolive bzw. dem Steckphantom vorhandenen Zugfäden gleichzeitig gespannt werden können. Dadurch ist der Aufwand zum Fixieren und Spannen beider Zugfäden gegenüber den bekannten Vorrichtungen wesentlich verringert, weil mit dem einen Fixierelement beide Zugfäden gleichzeitig gespannt werden können, nachdem sie am Fixierelement fixiert wurden. Des Weiteren ermöglicht diese erfindungsgemäße Ausgestaltung der Vorrichtung auch insbesondere ein gleichmäßiges Spannen beider Zugfäden, was bei den bekannten Vorrichtungen nur erschwert möglich ist.

In einer Ausgestaltung der zuvor genannten Maßnahme ist am Grundkörper für den weiteren Zugfaden eine Zuführstelle vorhanden, die in Bezug auf das Fixierelement der Zuführstelle für den anderen Zugfaden gegenüberliegend angeordnet ist.

Hierbei ist nun insbesondere in Verbindung mit dem vorgesehenen Spannmechanismus, der durch die Drehbarkeit des Fixierelements gegeben ist, besonders von Vorteil, dass beide Zugfäden von etwa gegenüberliegenden Seiten des Grundkörpers von ihren Zuführstellen auf das Fixierelement zulaufen können, wodurch die Zugfäden mit einem Abstand zueinander in die Vorrichtung laufen, der etwa dem Abstand der Inzision in der Bauchdecke der Patientin entspricht, aus denen die Fäden aus dem Bauch herausgeführt sind. Auf diese Weise ist die Zugkraftrichtung optimiert.

In einer weiteren bevorzugten Ausgestaltung ist für den weiteren Zugfaden eine weitere Feder am Grundkörper angeordnet, die der Feder für den anderen Zugfaden in Bezug auf das Fixierelement gegenüberliegend angeordnet ist.

Durch diese Maßnahme ergibt sich insgesamt eine bezüglich des Fixierelements spiegelsymmetrische Anordnung der Federn und Zuführstellen der beiden Zugfäden, wodurch die erfindungsgemäße Vorrichtung kompakt aufgebaut sein kann, d.h. einen geringen Platz auf dem Körper der Patientin einnimmt. Darüber hinaus ist die Zugkraftverteilung der beiden Zugfäden bezüglich des Fixierelements ebenfalls spiegelsymmetrisch, wodurch die Lage der Vorrichtung auf dem Körper der Patientin stabil ist.

In einer weiteren bevorzugten Ausgestaltung ist die Vorrichtung sterilisierbar, insbesondere autoklavierbar.

Hierbei ist von Vorteil, dass die Vorrichtung wiederverwendbar ist, wodurch die Anschaffungskosten einer solchen Vorrichtung in Bezug auf die Nutzungsdauer verringert sind.

Dabei kann auch vorgesehen sein, dass die funktionellen Teile, wie das Fixierelement, beispielsweise die Federn und das Rastgesperre, vom Grundkörper abnehmbar sind, wodurch die Reinigbarkeit der erfindungsgemäßen Vorrichtung weiter verbessert ist.

Weitere Vorteile und Merkmale ergeben sich aus der nachfolgenden Beschreibung und der beigefügten Zeichnung.

Ein Ausführungsbeispiel der Erfindung ist in der Zeichnung dargestellt und wird mit Bezug auf diese hiernach näher beschrieben. Es zeigen:
- Fig. 1: eine Vorrichtung zum Fixieren und Spannen zumindest eines Zugfadens für das Anlegen einer Neovagina in einer perspektivischen Ansicht von oben;
- Fig. 2: eine Draufsicht auf die Vorrichtung in Fig. 1, teilweise unter Weglassung von Teilen der Vorrichtung;
- Fig. 3: die Vorrichtung in Fig. 1 unter weiterer Weglassung von Teilen in einer perspektivischen Ansicht von oben;
- Fig. 4: ein Detail der Vorrichtung in Fig. 1 in Seitenansicht und gegenüber Fig. 1 vergrößertem Maßstab;
- Fig. 5: eine Draufsicht auf die Vorrichtung in Fig. 1, wobei zusätzlich zwei Zugfäden eingezeichnet sind; und

- Fig. 6: eine schematische Darstellung, die die Verwendung der Vorrichtung in Fig. 1 beim Anlegen einer Neovagina zeigt.

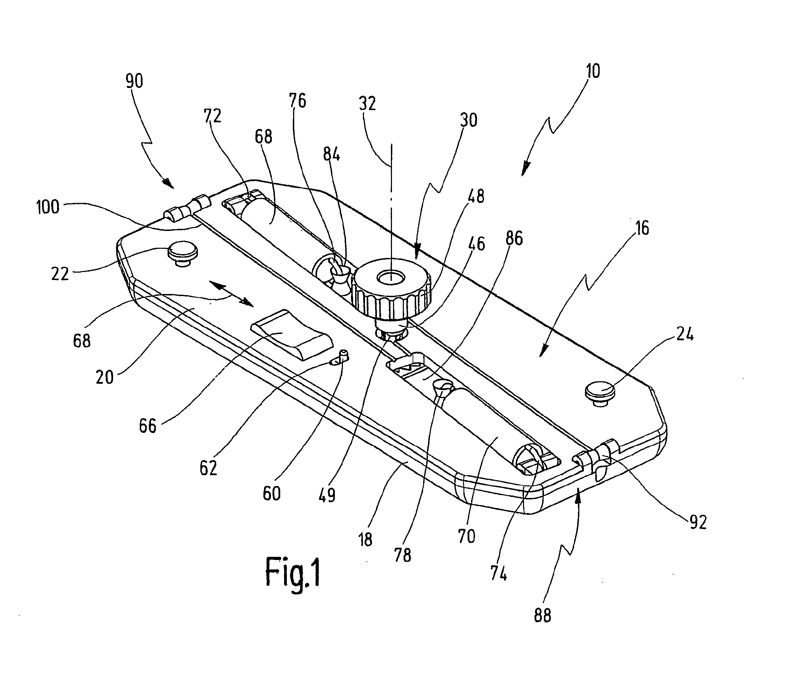

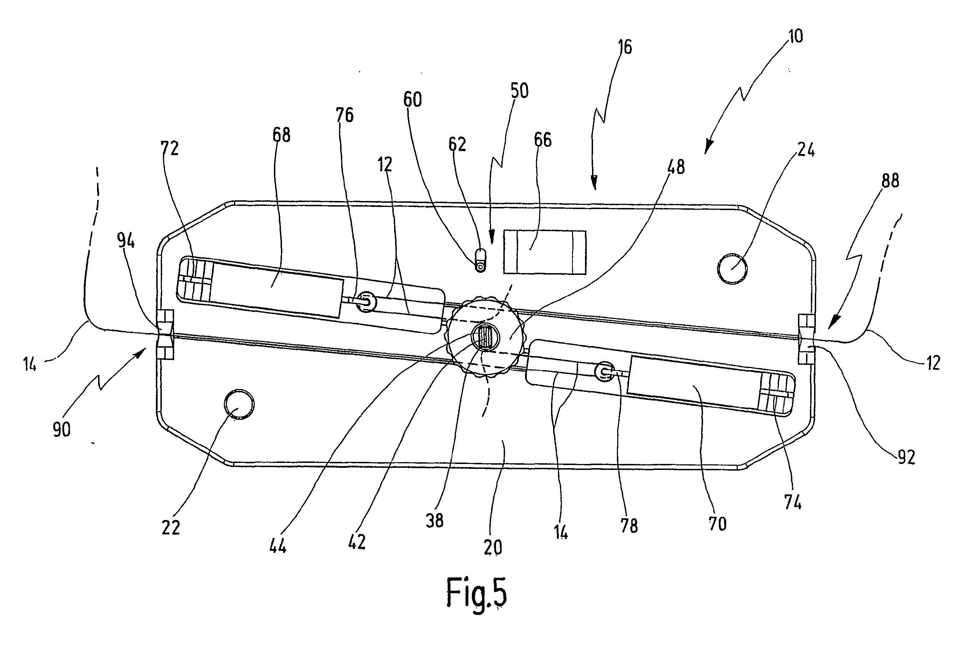

In Fig. 1 bis 6 ist eine mit dem allgemeinen Bezugszeichen 10 zum Fixieren und Spannen zumindest eines, im vorliegenden Ausführungsbeispiel zweier Zugfäden 12 und 14 (vgl. Fig. 5) für das Anlegen einer Neovagina dargestellt. Bevor das Fixieren und Spannen der beiden Zugfäden 12 und 14 beschrieben wird, wird zunächst die Vorrichtung 10 hinsichtlich ihres Aufbaus näher beschrieben.

Die Vorrichtung 10 weist einen Grundkörper 16 auf, der ohne Beschränkung der Allgemeinheit im Wesentlichen die Form eines Rechteckes aufweist. Die Ecken des Rechtecks sind, wie aus den Figuren ersichtlich ist, abgeschrägt, und die Seitenränder des Grundkörpers 16 sind vorzugsweise abgerundet. Der Grundkörper 16 ist im Wesentlichen planeben mit geringer Dicke ausgebildet, die Unterseite (nicht dargestellt) ist glatt und eben.

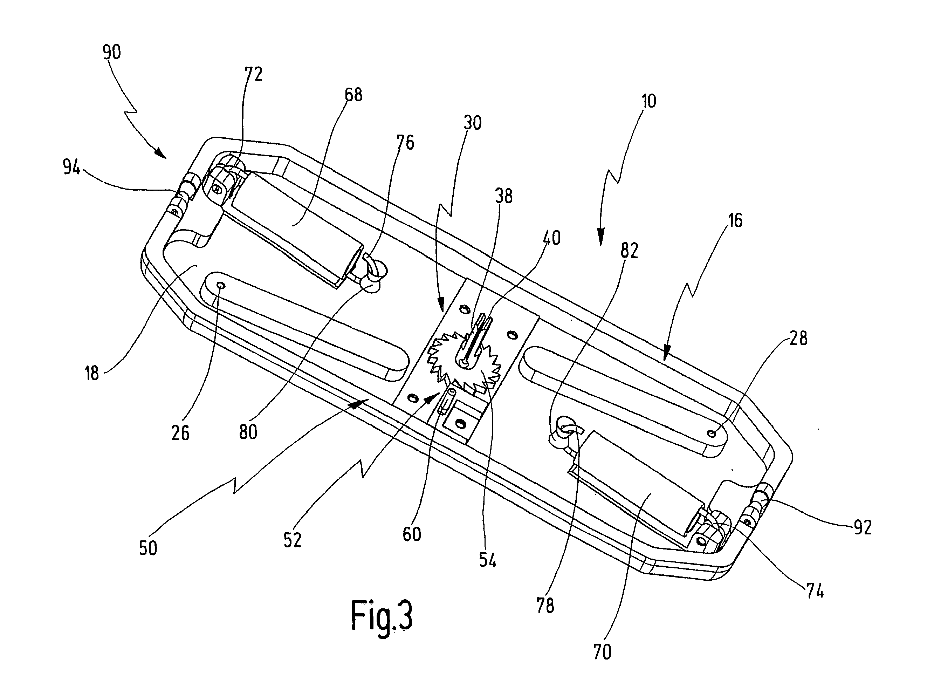

Der Grundkörper 16 ist durch eine Bodenplatte 18 und eine Deckplatte 20 gebildet, wobei die Deckplatte 20 von der Bodenplatte 18 abnehmbar ist. Fig. 3 zeigt beispielsweise die Vorrichtung 10 mit abgenommener Deckplatte 20.

Die Deckplatte 20 ist mittels zweier von Hand ohne Werkzeug betätigbarer Schrauben 22 und 24 (Fig. 1) an der Bodenplatte 18 befestigt, wobei in der Bodenplatte 18 entsprechende Gewindebohrungen 26 und 28 für die Schrauben 22 und 24 vorhanden sind.

Insbesondere die Bodenplatte 18, vorliegend auch die Deckplatte 20, sind aus einem biokompatiblen Material gebildet, so dass die Vorrichtung 10 bei einer Patientin keine Hautunverträglichkeiten hervorruft.

Die Vorrichtung 10 ist insgesamt sterilisierbar, insbesondere autoklavierbar und kann somit mehrfach für verschiedene Patientinnen verwendet werden.

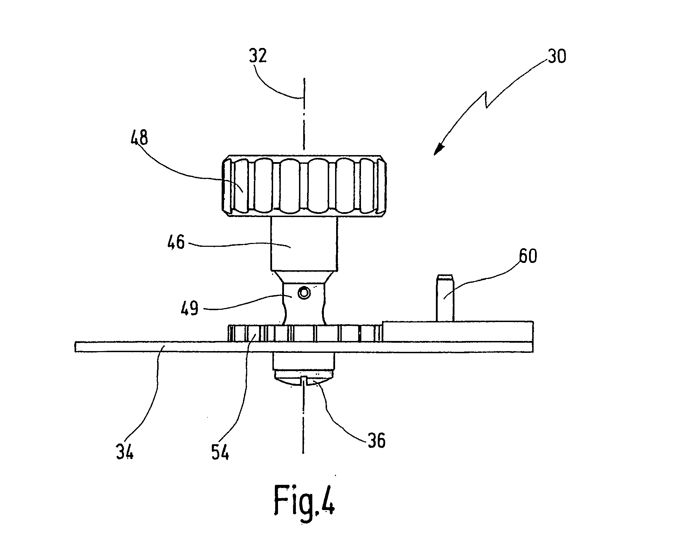

An dem Grundkörper 16 ist ein Fixierelement 30 angeordnet. In Fig. 4 ist das Fixierelement 30 vergrößert in Seitenansicht dargestellt.

Das Fixierelement 30 ist um eine Drehachse 32 drehbar an einer mit der Bodenplatte 18 verbundenen Zwischenplatte 34 mittels einer Schraube 36 befestigt (vgl. Fig. 4). Die Schraube 36 ist von der Unterseite der Bodenplatte 18 her zugänglich.

Das Fixierelement 30 weist einen sich von der Bodenplatte 18 aus gesehen nach oben erstreckenden geschlitzten Abschnitt 38 auf, der einen Schlitz 40 (vgl. Fig. 3) aufweist. Der Schlitz 40 dient der Aufnahme eines Abschnittes 42 bzw. 44 (vgl. Fig. 5) der Zugfäden 12 und 14, wobei die Abschnitte 42 und 44 im Wesentlichen die Enden der Zugfäden 12 und 14 bilden.

Zum Festklemmen der Abschnitte 42 und 44 der Zugfäden 12 und 14 in dem Schlitz 40 weist das Fixierelement 30 weiterhin eine Hülse 46 auf, die auf den geschlitzten Abschnitt 38 zum Festklemmen der Zugfäden 12 und 14, genauer gesagt der Abschnitte 42 und 44 der Zugfäden 12 und 14, auf den geschlitzten Abschnitt 38 aufsteckbar und auf diesem drehfest mit dem geschlitzten Abschnitt 38 verrastbar ist.

Die Hülse 46 weist weiterhin einstückig mit ihr verbunden ein Stellrad 48 auf, dessen Rand zur besseren Griffigkeit vorzugsweise strukturiert, beispielsweise gerändelt ist, wobei das Stellrad 48 als Betätigungselement zum Drehen des Fixierelements 30 dient.

Wie später noch beschrieben wird, können beide Zugfäden 12 und

14 durch Einlegen der Abschnitte 42 und 44 in den Schlitz 40 und Aufsetzen der Hülse 46 an dem Fixierelement 30 fixiert und danach gleichzeitig und gleichmäßig durch Drehen des Fixierelements 30 um die Drehachse 32 gespannt werden. Durch Drehen des Fixierelements 38 wickeln sich die Zugfäden 12 und 14 um einen Abschnitt 49 des geschlitzten Abschnitts 38 auf, wodurch sie gespannt werden. Die Drehrichtung des Fixierelements 30 zum Spannen der Zugfäden 12 und 14 ist im vorliegenden Ausführungsbeispiel der Uhrzeigersinn. Das Fixierelement 30 ist zwar auch im Gegenuhrzeigersinn drehbar, jedoch ist das Fixierelement 30 zumindest in dieser Drehrichtung, die also der Drehrichtung zum Spannen des Zugfadens entgegengerichtet ist, feststellbar.

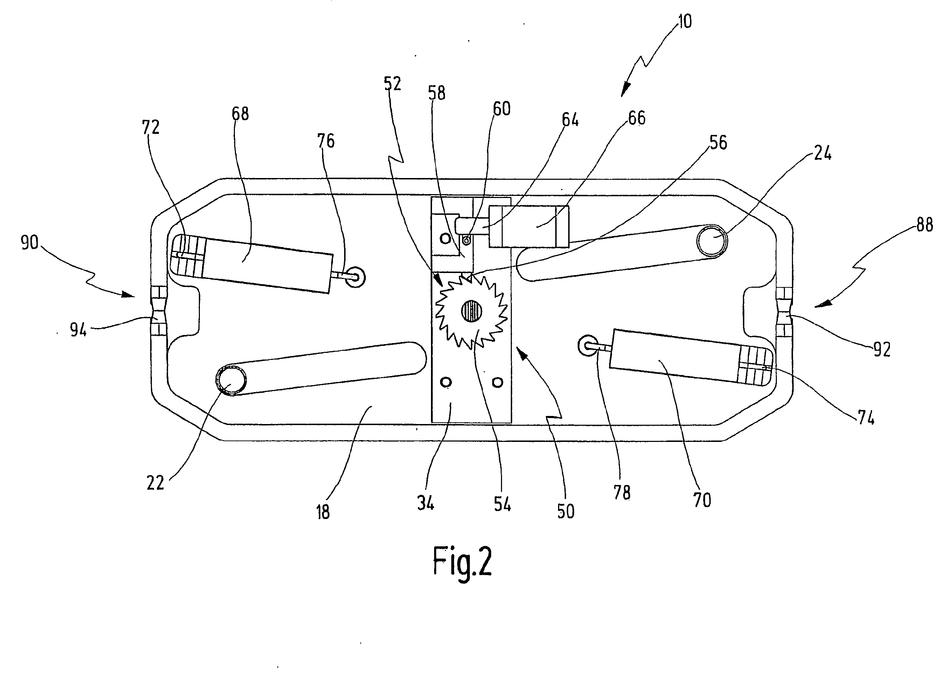

Dazu weist die Vorrichtung 10 eine Feststelleinrichtung 50 auf, die in Form eines Rastgesperres 52 ausgebildet ist, das am deutlichsten in Fig. 2 und Fig. 3 zu erkennen ist.

Das Fixierelement 30, das in Fig. 2 und 3 ohne die Hülse 46 und dem Stellrad 48 dargestellt ist, weist ein umfänglich gezahntes Rad 54 auf, sowie ein mit dem gezahnten Rad 54 zusammenwirkendes erstes Sperrelement 56, das in Form eines Stiftes oder Zapfens ausgebildet ist, der so bemessen und geformt ist, dass er im Wesentlichen formschlüssig zwischen jeweils zwei Zähne des gezahnten Rades 54 eingreifen kann, wie in Fig. 2 dargestellt ist.

Das gezahnte Rad 54 ist mit dem geschlitzten Abschnitt 38 des Fixierelements 30 drehfest verbunden.

Das Sperrelement 56 ist in einer Führung 58 längs verschiebbar angeordnet und fest mit einem Betätigungselement 60 zum In-und-Außer-Eingriff-Bringen des Sperrelements 56 mit dem gezahnten Rad 54 verbunden.

Das Betätigungselement 60 ragt im zusammengebauten Zustand der Vorrichtung 10 durch die Deckplatte 20 durch ein Langloch 62 in derselben hindurch.

Das Rastgesperre 52 ist ferner gegen ein unerwünschtes Entsperren verriegelbar. Dazu ist das erste Sperrelement 56, das wie bereits erwähnt in seiner Längsrichtung beweglich ist, feststellbar, wenn es mit der Verzahnung des gezahnten Rades 54 in Eingriff steht, wie in Fig. 2 dargestellt ist. Zum Feststellen des ersten Sperrelements 56 ist ein zweites Sperrelement 64 vorgesehen, das in den Bewegungsweg des ersten Sperrelements 56 verstellbar ist. Das zweite Sperrelement 64 ist in Form eines Zapfens ausgebildet, der mit einem Betätigungselement 66 fest verbunden ist, um das zweite Sperrelement 64 in den Bewegungsweg des ersten Sperrelements 56 bzw. aus diesem heraus zu verschieben. Das Betätigungselement 66 ist in Form eines Schiebers ausgebildet, der in Richtungen eines Doppelpfeils 68 verschiebbar ist, d.h. das Betätigungselement 66 ist in Richtungen quer zur Beweglichkeit des ersten Sperrelements 56 beweglich.

In der in Fig. 2 dargestellten Betriebsstellung des zweiten Sperrelements 64 blockiert es die Beweglichkeit des Betätigungselements 60 und damit des ersten Sperrelements 56, das somit in der in Fig. 2 dargestellten Betriebsstellung des zweiten Sperrelements 64 sicher im Eingriff mit dem gezahnten Rad 54 gehalten wird, wodurch das Fixierelement 30 sogar vollständig drehfest gehalten wird.

Dem Fixierelement 30 ist für jeden Zugfaden 12 und 14 jeweils eine Feder zugeordnet, und zwar für den Zugfaden 12 eine Feder 68 und für den Zugfaden 14 eine Feder 70. Die Federn 68 und 70 sind als Schraubenfedern ausgebildete Zugfedern, die im gezeigten Ausführungsbeispiel jeweils von einer Hülse umgeben sind.

Die Feder 68 ist mit einem ersten Ende 72 am Grundkörper 16, genauer gesagt an dessen Bodenplatte 18, befestigt, und zwar im gezeigten Ausführungsbeispiel gelenkig befestigt. Entsprechend ist die Feder 70 mit einem ersten Ende 74 am Grundkörper 16, d.h. an der Bodenplatte 18, gelenkig befestigt.

Ein zweites Ende 76 der Feder 68 ist zur beweglichen Verbindung mit dem Zugfaden 12 ausgebildet, und ein zweites Ende 78 der Feder 70 ist zur beweglichen Verbindung mit dem Zugfaden 14 ausgebildet. Des Weiteren wird der Zugfaden 12 an dem zweiten Ende 76 der Feder 68 zum Fixierelement 30 hin umgelenkt, und der Zugfaden 14 wird an dem zweiten Ende 78 der Feder 70 ebenfalls zum Fixierelement 30 hin umgelenkt, wie in Fig. 5 dargestellt ist. An den zweiten Enden 76 und 78 ist jeweils eine bewegliche, d.h. drehbewegliche, Führungsrolle 80 bzw. 82 angeordnet.

Wie insbesondere aus Fig. 1 hervorgeht, sind in der Deckplatte 20 des Grundkörpers 16 langlochförmige Aussparungen 84 und 86 vorhanden, in denen die Feder 68 bzw. 70 von außen zugänglich zu liegen kommen, wenn die Deckplatte 20 und die Bodenplatte 18 zusammengefügt sind. Die Aussparungen 84 und 86 sind entsprechend der möglichen Dehnung der Federn 68 und 70 länger als die Federn 68 und 70 in deren entspannten Zustand ausgebildet.

Sowohl für den Zugfaden 12 als auch für den Zugfaden 14 ist jeweils eine Zuführstelle am Grundkörper 16 vorgesehen, und zwar für den Zugfaden 12 (vgl. Fig. 5) eine Zuführstelle 88 und für den Zugfaden 14 eine Zuführstelle 90.

Die Zuführstellen 88 und 90, die ein definiertes Zuführen der Zugfäden 12 und 14 in die Vorrichtung 10 und somit eine definierte Zugkraftrichtung für die Zugfäden 12 und 14 gewährleisten, weisen jeweils eine bewegliche, d.h. drehbewegliche Führungsrolle 92, 94 auf, wobei die Zugfäden 12 bzw. 14 über den Führungsrollen 92 bzw. 94 geführt werden, wie dies aus Fig. 5 hervorgeht. Die Führungsrollen 92 und 94 sind an der Bodenplatte 18 des Grundkörpers 16 befestigt (vgl. Fig. 3). Die Zuführstellen 88 und 90 sind nun bezüglich der Federn 68 und 70 und dem Fixierelement 30 so angeordnet, dass der Zugfaden 12 von der Zuführstelle 88 zunächst an dem Fixierelement 30 vorbei zu dem zweiten Ende 76 der Feder 68 verläuft, dort um etwa 180° umgelenkt und anschließend zum Fixierelement 30 verläuft. Das Gleiche gilt für den Zugfaden 14, der von der Zuführstelle 90 zunächst an dem Fixierelement 30 vorbei zu dem zweiten Ende 78 der Feder 70 verläuft, dort um etwa 180° umgelenkt und schließlich zum Fixierelement 30 geführt wird.

Die gesamte Anordnung aus den Zuführstellen 88, 90, den Federn 68, 70 und dem Fixierelement 30 ist im Wesentlichen spiegelsymmetrisch bezüglich dem Fixierelement 30 gestaltet, d.h. die Zuführstellen 88 und 90 liegen sich bezüglich dem Fixierelement 30 diametral gegenüber, ebenso die Federn 68 und 70.

Mittels des nur einen Fixierelements 30 können nun beide Zugfäden 12 und 14 fixiert und gleichzeitig und vor allem gleichmäßig mit geringem Handhabungsaufwand gespannt werden, indem nämlich einfach nur nach Festlegung der Abschnitte 42 und 44 an dem Fixierelement 30 das Stellrad 48 im Uhrzeigersinn gedreht wird.

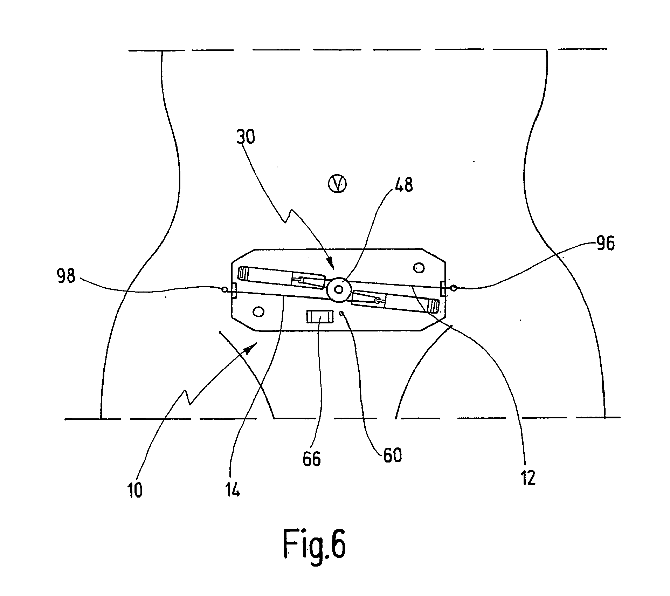

Nachfolgend wird zusätzlich mit Bezug auf Fig. 6 die Verwendung bei einem Verfahren zum Anlegen einer Neovagina und die Bedienung der Vorrichtung 10 beschrieben.

Bei dem Verfahren zum Anlegen einer Neovagina werden die beiden Zugfäden 12 und 14, die mit einer nicht dargestellten Kunststoffolive oder einem nicht dargestellten Steckphantom verbunden sind, die bzw. das auf das Vaginalgrübchen einen Druck ausüben soll, um die Neovagina zu bilden, intraperitoneal von der Kunststoffolive bzw. dem Steckphantom bis zu zwei Inzisionen 96 und 98 geführt und dort aus dem Körper herausgeführt.

Die Vorrichtung 10 wird oberhalb des Schambereiches auf dem Bauch der Patientin positioniert, so dass die Zuführstellen 88, 90 etwa auf Höhe der Inzisionen 96, 98 liegen. Die Fadenenden der Zugfäden 12 und 14 werden nun über die Zuführstelle 88 bzw. die Zuführstelle 90 in die Vorrichtung eingeführt und dann, wie zuvor beschrieben, um das zweite Ende 76 der Feder 68 bzw. das zweite Ende 78 der Feder 70 gelegt und von dort zum Fixierelement 30 geführt. Die Enden bzw. Abschnitte 42 und 44 der Zugfäden 12 und 14 werden dann in den Schlitz 40 des geschlitzten Abschnittes 38 des Fixierelementes eingelegt, wobei die Hülse 46 mit dem Stellrad 48 von dem geschlitzten Abschnitt 38 abgezogen ist. Anschließend wird die Hülse 46 mit dem Stellrad 48 auf den geschlitzten Abschnitt 38 aufgesteckt und drehfest verrastet.

Nun wird oder es wurde bereits zuvor das Rastgesperre 52 entriegelt, indem das Betätigungselement 60 für das erste Sperrelement 56 zurückgezogen wurde, so dass das erste Sperrelement 56 mit dem gezahnten Rad 54 außer Eingriff kommt. Um das Betätigungselement 60 zurückziehen zu können, muss das Betätigungselement 66 für das zweite Sperrelement 64 ebenfalls zurück verschoben worden sein, und zwar in der Darstellung in Fig. 1 nach links.

Nunmehr lassen sich die Zugfäden 12 und 14 durch Drehen des Fixierelements 30 im Uhrzeigersinn spannen, bis eine gewünschte Zugspannung erreicht ist. Wenn die geeignete und gewünschte Zugspannung eingestellt ist, wird das erste Sperrelement mittels des Betätigungselements 60 mit dem gezahnten Rad 54 in Eingriff gebracht, und zur Verriegelung des Rastgesperres 52 wird dann das Betätigungselement 66 für das zweite Sperrelement 64 (in Fig. 1 nach rechts) verschoben, wodurch das Fixierelement 30 gegen eine Drehung entgegen der Spannrichtung gesichert ist.

Zum Nachspannen oder auch zum Entspannen der Zugfäden 12 und 14, falls diese zunächst zu stark gespannt wurden, muss lediglich mittels der Betätigungselemente 66 und 60 das Rastgesperre entriegelt werden, wonach durch Drehen des Fixierelements 30 im Uhrzeigersinn (Spannen) bzw. im Gegenuhrzeigersinn (Entspannen) die jeweils optimale Zugkraft der Zugfäden 12 und 14 eingestellt werden kann, wobei dies für die Zugfäden 12 und 14 gleichzeitig und somit gleichmäßig erfolgt.

Wie in Fig. 1 und 5 dargestellt ist, sind in der Deckplatte 20 des Grundkörpers 16 Rillen 100 ausgespart, in denen die Zugfäden 12 und 14 versenkt sind und somit nicht die Gefahr eines Hängenbleibens an den Zugfäden 12 und 14 besteht.

## Patentansprüche

1. Vorrichtung zum Fixieren und Spannen zumindest eines Zugfadens (12, 14) für das Anlegen einer Neovagina, mit einem Grundkörper (16), an dem zumindest ein Fixierelement (30) zum Fixieren des zumindest einen Zugfadens (12, 14) unter Spannung angeordnet ist, wobei das Fixierelement (30) zum Spannen des zumindest einen Zugfadens (12, 14) um eine Drehachse (32) drehbar ist, wobei eine Feststelleinrichtung (50) für das Fixierelement (30) vorhanden ist, mittels der das Fixierelement (30) zumindest in einer Drehrichtung, die der Drehrichtung zum Spannen des zumindest einen Zugfadens (12, 14) entgegengerichtet ist, feststellbar ist, **dadurch gekennzeichnet, dass** dem Fixierelement (30) eine Feder (68, 70) zugeordnet ist, deren erstes Ende (72, 74) am Grundkörper (16) festgelegt ist, und mit deren zweitem Ende (76, 78 der Zugfaden (12, 14) beweglich verbindbar ist, so daß der Zugfaden (12, 14) zum Fixierelement (30) hin umgelenkt wird.

2. Vorrichtung nach Anspruch 1, **dadurch gekennzeichnet, dass** die Feststelleinrichtung ein Rastgesperre (52) zum Feststellen des Fixierelements (30) in der zumindest einen Drehrichtung aufweist.

3. Vorrichtung nach Anspruch 2, **dadurch gekennzeichnet, dass** das Rastgesperre (52) gegen unerwünschtes Entsperren verriegelbar ist.

4. Vorrichtung nach einem der Ansprüche 1 bis 3, **dadurch gekennzeichnet, dass** das Fixierelement (30) ein umfänglich gezahntes Rad (54) aufweist, das zum Feststellen des Fixierelements (30) mit einem mit der Verzahnung des Rads (54) in Eingriff bringbaren ersten Sperrelement (56) zusammenwirkt.

5. Vorrichtung nach Anspruch 4, **dadurch gekennzeichnet, dass** das erste Sperrelement (56) feststellbar ist, wenn es mit der Verzahnung des Rades (54) in Eingriff steht.

6. Vorrichtung nach Anspruch 5, **dadurch gekennzeichnet, dass** zum Feststellen des ersten Sperrelements (56) ein zweites Sperrelement (64) vorhanden ist, das in den Bewegungsweg des ersten Sperrelements (56) verstellbar ist.

7. Vorrichtung nach einem der Ansprüche 1 bis 6, **dadurch gekennzeichnet, dass** das Fixierelement (30) einen geschlitzten Abschnitt (38) zur Aufnahme eines Abschnitts (42, 44) des zumindest einen Zugfadens (12, 14), und eine Hülse (46) zum Festklemmen des Zugfadens (12, 14) am Fixierelement (30) aufweist.

8. Vorrichtung nach Anspruch 7, **dadurch gekennzeichnet, dass** die Hülse (46) ein Stellrad (48) zum Drehen des Fixierelements (30) aufweist.

9. Vorrichtung nach einem der Ansprüche 1 bis 8, **dadurch gekennzeichnet, dass** am zweiten Ende (76, 78) der Feder (68, 70) eine bewegliche Führungsrolle (80, 82) als Umlenkung angeordnet ist.

10. Vorrichtung nach einem der Ansprüche 1 bis 9, **dadurch gekennzeichnet, dass** am Grundkörper (16) eine Zuführstelle (88, 90) für den Zugfaden (12, 14) vorhanden ist.

11. Vorrichtung nach Anspruch 10, **dadurch gekennzeichnet, dass** die Zuführstelle (88, 90) bezüglich der Feder (68, 70) und dem Fixierelement (30) so angeordnet ist, dass der Zugfaden (12, 14) von der Zuführstelle (88, 90) zunächst an dem Fixierelement (30) vorbei zu dem zweiten Ende (78, 80) der Feder (68, 70) und von dort zum Fixierelement (30) verläuft.

12. Vorrichtung nach Anspruch 11, **dadurch gekennzeichnet, dass** die Zuführstelle (88, 90) bezüglich der Feder (68, 70) und dem Fixierelement (30) so angeordnet ist, dass der Zugfaden (12, 14) an dem zweiten Ende (78, 80) um etwa 180° zu dem Fixierelement (30) hin umgelenkt wird.

13. Vorrichtung nach einem der Ansprüche 10 bis 12, **dadurch gekennzeichnet, dass** an der Zuführstellen (88, 90) eine bewegliche Führungsrolle (92, 94) vorhanden ist.

14. Vorrichtung nach einem der Ansprüche 1 bis 13, **dadurch gekennzeichnet, dass** das Fixierelement (30) zum gemeinsamen Fixieren und Spannen des zumindest einen Zugfadens (12) und zumindest eines weiteren zugfadens (14) ausgelegt ist.

15. Vorrichtung nach Anspruch 14 und einem der Ansprüche 10 bis 13, **dadurch gekennzeichnet, dass** am Grundkörper (16) für den weiteren Zugfaden (14) eine Zuführstelle (90) vorhanden ist, die in bezug auf das Fixierelement (30) der Zuführstelle (88) für den anderen Zugfaden (12) gegenüberliegend angeordnet ist.

16. Vorrichtung nach Anspruch 14 oder 15, **dadurch gekennzeichnet, dass** für den weiteren Zugfaden (14) eine weitere Feder (70) am Grundkörper (16) angeordnet ist, die der Feder (68) für den anderen Zugfaden (12) in bezug auf das Fixierelement (30) gegenüberliegend angeordnet ist.

17. Vorrichtung nach einem der Ansprüche 1 bis 16, **dadurch gekennzeichnet, dass** sie sterilisierbar, insbesondere autoklavierbar ist.

## Claims

1. A device for fixing and tensioning at least one pulling thread (12, 14) for applying a neovagina, comprising a base body (16) on which at least one fixing element (30) is arranged for fixing the at least one pulling thread (12, 14) with tensioning, wherein the fixing element (30) is able to turn about an axis of rotation (32) in order to tension the at least one pulling thread (12, 14), and a locking device (50) is provided for the fixing element (30), by means of which locking device (50) the fixing element (30) can be locked at least in a direction of rotation opposite to the direction of rotation for tensioning the at least one pulling thread (12, 14), **characterized in that** the fixing element (30) is assigned a spring whose first end (72, 74) is secured on the base body (16), and whose second end (76, 78) is configured for movable connection of the pulling thread (12, 14) thereto so that the pulling thread (12, 14) is deflected toward the fixing element (30).

2. The device of claim 1, **characterized in that** the locking device has a latching mechanism (52) for locking the fixing element (30) in the at least one direction of rotation.

3. The device of claim 2, **characterized in that** the latching mechanism (52) can be secured against undesired unlocking.

4. The device of anyone of claims 1 through 3, **characterized in that** the fixing element (30) has a circumferentially toothed wheel (54) which, in order to lock the fixing element (30), interacts with a first catch element (56) that can be brought into engagement with the toothing of the wheel (54).

5. The device of claim 4, **characterized in that** the first catch element (56) can be locked when it is in engagement with the toothing of the wheel (54).

6. The device of claim 5, **characterized in that**, in order to lock the first catch element (56), a second catch element (64) is provided which can be shifted into the path of movement of the first catch element (56).

7. The device of anyone of claims 1 through 6, **characterized in that** the fixing element (30) has a slotted portion (38) for receiving a portion (42, 44) of the at least one pulling thread (12, 14), and a sleeve (46) for clamping the pulling thread (12, 14) on the fixing element (30).

8. The device of claim 7, **characterized in that** the sleeve (46) has a control wheel (48) for turning the fixing element (30).

9. The device of anyone of claims 1 through 8, **characterized in that** a movable guide roller (80, 82) as deflection is arranged at the second end (76, 78) of the spring (68, 70).

10. The device of anyone of claims 1 through 9, **characterized in that** a feed point (88, 90) for the pulling thread (12, 14) is present on the base body (16).

11. The device of claim 10, **characterized in that** the feed point (88, 90) is arranged in relation to the spring (68, 70) and to the fixing element (30) in such a way that the pulling thread (12, 14) first runs from the feed point (88, 90) past the fixing element (30) to the second end (78, 80) of the spring (68, 70) and from there to the fixing element (30).

12. The device of claim 11, **characterized in that** the feed point (88, 90) is arranged in relation to the spring (68, 70) and to the fixing element (30) in such a way that the pulling thread (12, 14) is deflected at the second end (78, 80) through approximately 180° toward the fixing element (30).

13. The device of anyone of claims 10 through 12, **characterized in that** a movable guide roller (92, 94) is present at the feed point (88, 90).

14. The device of anyone of claims 1 through 13, **characterized in that** the fixing element (30) is designed for jointly fixing and tensioning the at least one pulling thread (12) and at least one further pulling thread (14).

15. The device of claim 14 and of anyone of claims 10 through 13, **characterized in that** a feed point (90) for the further pulling thread (14) is provided on the base body (16), which feed point (90) is arranged, in relation to the fixing element (30), opposite the feed point (88) for the other pulling thread (12).

16. The device of claim 14 or 15, **characterized in that** a further spring (70) for the further pulling thread (14) is arranged on the base body (16) and, in relation to the fixing element (30), is arranged opposite the spring (68) for the other pulling thread (12).

17. The device of anyone of claims 1 through 16, **characterized in that** it can be sterilized, in particular autoclaved.

## Revendications

1. Dispositif de blocage et de tension d'au moins d'un fil de traction (12, 14) pour l'application d'un néovagin, avec un corps de base (16), sur lequel est disposé au moins un élément de fixation (30) pour la fixation de l'au moins un fil de traction (12, 14) sous tension, l'élément de fixation (30) pouvant être tourné pour la tension de l'au moins un fil de traction (12, 14) autour d'un axe de rotation (32), un dispositif de blocage (50) pour l'élément de fixation (30) étant présent, au moyen duquel l'élément de fixation (30) peut être bloqué au moins dans un sens de rotation qui est contraire au sens de rotation pour la tension de l'au moins un fil de traction (12, 14), **caractérisé en ce qu'**à l'élément de fixation (30) est associé un ressort (68, 70), dont la première extrémité (72, 74) est fixée sur le corps de base (16), et à la seconde extrémité (76, 78) duquel, le fil de traction (12, 14) peut être relié de manière mobile de sorte que le fil de traction (12, 14) soit dévié vers l'élément de fixation.

2. Dispositif selon la revendication 1, **caractérisé en ce que** le dispositif de blocage présente un dispositif d'encliquetage (52) pour le blocage de l'élément de fixation (30) dans l'au moins un sens de rotation.

3. Dispositif selon la revendication 2, **caractérisé en ce que** le dispositif d'encliquetage (52) peut être verrouillé contre tout déblocage non souhaité.

4. Dispositif selon l'une quelconque des revendications 1 à 3, **caractérisé en ce que** l'élément de fixation (30) présente une roue (54) dentée sur la périphérie qui coopère pour le blocage de l'élément de fixation (30) avec un premier élément de blocage (56) pouvant être amené en prise avec la denture de la roue (54).

5. Dispositif selon la revendication 4, **caractérisé en ce que** le premier élément de blocage (56) peut être bloqué s'il est en prise avec la denture de la roue (54).

6. Dispositif selon la revendication 5, **caractérisé en ce que** pour le blocage du premier élément de blocage (56), un second élément de blocage (64) est présent, lequel peut être réglé dans la course de déplacement du premier élément de blocage (56).

7. Dispositif selon l'une quelconque des revendications 1 à 6, **caractérisé en ce que** l'élément de fixation (30) présente une section (38) fendue pour la réception d'une section (42, 44) de l'au moins un fil de traction (12, 14) et une douille (46) pour le serrage à bloc du fil de traction (12, 14) sur l'élément de fixation (30).

8. Dispositif selon la revendication 7, **caractérisé en ce que** la douille (46) présente une roue de réglage (48) pour la rotation de l'élément de fixation (30).

9. Dispositif selon l'une quelconque des revendications 1 à 8, **caractérisé en ce que** sur la seconde extrémité (76, 78) du ressort (68, 70) est disposé un rouleau de guidage (80, 82) servant de déviation.

10. Dispositif selon l'une quelconque des revendications 1 à 9, **caractérisé en ce que** sur le corps de base (16) se trouve un point d'alimentation (88, 90) pour le fil de traction (12, 14).

11. Dispositif selon la revendication 10, **caractérisé en ce que** le point d'alimentation (88, 90) est disposé par rapport au ressort (68, 70) et à l'élément de fixation (30) de sorte que le fil de traction (12, 14) s'étende tout d'abord du point d'alimentation (88, 90) devant l'élément de fixation (30) jusqu'à la seconde extrémité (78, 80) du ressort (68, 70) et de là jusqu'à l'élément de fixation (30).

12. Dispositif selon la revendication 11, **caractérisé en ce que** le point d'alimentation (88, 90) est disposé par rapport au ressort (68, 70) et à l'élément de fixation (30) de sorte que le fil de traction (12, 14) soit dévié sur la seconde extrémité (78, 80) d'environ 180° vers l'élément de fixation (30).

13. Dispositif selon l'une quelconque des revendications 10 à 12, **caractérisé en ce qu'**un rouleau de guidage (92, 94) mobile se trouve sur le point d'alimentation (88, 90).

14. Dispositif selon l'une quelconque des revendications 1 à 13, **caractérisé en ce que** l'élément de fixation (30) est configuré pour la fixation et la tension communes de l'au moins un fil de traction (12) et au moins d'un autre fil de traction (14).

15. Dispositif selon la revendication 14 et l'une quelconque des revendications 10 à 13, **caractérisé en ce que** sur le corps de base (16) pour l'autre fil de traction (14) se trouve un point d'alimentation (90) qui est disposé à l'opposé par rapport à l'élément de fixation (30) du point d'alimentation (88) pour l'autre fil de traction (12).

16. Dispositif selon la revendication 14 ou 15, **caractérisé en ce que** pour l'autre fil de traction (14), un autre ressort (70) est disposé sur le corps de base (16), lequel est disposé à l'opposé du ressort (68) pour l'autre fil de traction (12) par rapport à l'élément de fixation (30).

17. Dispositif selon l'une quelconque des revendications 1 à 16, **caractérisé en ce qu'**il peut être stérilisé, en particulier autoclavé.
